# EUROPEAN PATENT SPECIFICATION

(11) **EP 3 769 702 B1**
(45) Date of publication and mention of the grant of the patent: **12.07.2023**
(21) Application number: 18910592.7
(22) Date of filing: 21.09.2018
(51) Int. Cl.: A61B 17/34, A61M 25/06, A61M 29/00, A61B 90/00

(54) **DILATOR**
DILATATOR
DILATATEUR

(30) Priority: 23.03.2018 WO PCT/JP2018/011673
(43) Date of publication of application: 27.01.2021
(73) Proprietor: ASAHI INTECC CO., LTD., Seto-shi, Aichi 489-0071 (JP)
(72) Inventor: FUSEYA, Yukihiro, Seto-shi, Aichi 489-0071 (JP); MAKI, Hideaki, Seto-shi, Aichi 489-0071 (JP); TAKAHASHI, Daiki, Seto-shi, Aichi 489-0071 (JP); SAWAI, Akira, Seto-shi, Aichi 489-0071 (JP); TSUZUKU, Marina, Seto-shi, Aichi 489-0071 (JP)
(74) Representative: Prinz & Partner mbB
(86) International application number: PCT/JP2018/035089
(87) International publication number: WO 2019/181012

(56) References cited:
- JP-A- 2002 177 289
- JP-A- 2007 098 120
- JP-A- 2014 524 807
- JP-U- H0 490 355
- US-A1- 2002 077 655
- US-A1- 2009 005 814
- US-A1- 2012 029 281
- US-A1- 2012 116 350

## Description

### TECHNICAL FIELD

The present invention relates to a dilator.

### BACKGROUND ART

Dilators that enable treatment by enlarging a penetration-hole formed in the wall of a patient's digestive tract or the like are known. The distal end of the dilator is inserted into the penetration-hole formed in the wall, and the penetration-hole is expanded by pushing a tapered part into the penetration-hole. Such a dilator is disclosed in Patent Literature 1, for example.

Generic US 2002/0077655 A1, US 2012/0116350 A1, US 2012/0029281 A1 and US 2009/005814 A1 show dilators with spiral structures at their distal ends.

### CITATION LIST

### Patent Literature

Patent Literature 1: Japanese Unexamined Patent Application Publication No. 2008-11867

### SUMMARY OF INVENTION

### TECHNICAL PROBLEM

In the dilator mentioned above, a sufficient thrust could not be achieved with respect to the tapered part, which experiences an increased resistance when being pushed into a penetration-hole or constricted part, and in some cases the penetration-hole or constricted part could not be sufficiently expanded.

The present invention has an object of providing a dilator that can easily widen the diameter of a penetration-hole formed in the wall of a digestive tract or the like, and can suppress damage to the wall of the digestive tract or the like.

### SOLUTION TO PROBLEM

In order to achieve the object, a dilator according to an aspect of the present invention comprises a shaft in a hollow shape, and a grip part that is provided on a proximal end of the shaft. The shaft comprises a tapered part in which an outer diameter of a distal end is smaller than an outer diameter of a proximal end, and either a distal end part that is provided on a distal end side of the tapered part and which extends toward the distal end side and a proximal end part that is provided on a proximal end side of the tapered part and which extends toward the proximal end side, or the proximal end part that is provided on the proximal end side of the tapered part and which extends toward the proximal end side, without the distal end part. A spirally-arranged protruding portion is provided on an outer peripheral face of the shaft, and the spirally-arranged protruding portion has gaps in sections that are neighboring along an axis of the shaft. If the shaft does not include the distal end part, a pitch of sections of the spirally-arranged protruding portion provided on the tapered part, which are neighboring along the axis, is larger than the pitch of sections of the spirally-arranged protruding portion provided on the proximal end part, which are neighboring along the axis. If the shaft includes the distal end part, a pitch of sections of the spirally-arranged protruding portion provided on the tapered part, which are neighboring along the axis, is larger than the pitch of sections of the spirally-arranged protruding portion provided on the distal end part or the proximal end part, which are neighboring, respectively, along the axis.

Furthermore, if the shaft includes the distal end part, the pitch of sections of the spirally-arranged protruding portion provided on the tapered part, which are neighboring along a direction of the axis, may be larger than the pitch of sections of the spirally-arranged protruding portion provided on the distal end part, which are neighboring along the direction of the axis, and may also be larger than the pitch of sections of the spirally-arranged protruding portion provided on the proximal end part, which are neighboring along the direction of the axis.

Moreover, the shaft may include a first coil formed of a wire wound in a hollow shape.

In addition, the spirally-arranged protruding portion may include a second coil formed of a wire wound around onto the outer peripheral face of the shaft.

Also, the shaft may include a first coil formed of a wire wound around in a hollow shape, the spirally-arranged protruding portion may include a second coil formed of a wire wound around onto the outer peripheral face of the shaft, and the wire of the first coil and the wire of the second coil may be wound in mutually opposite directions.

### ADVANTAGEOUS EFFECTS OF INVENTION

According to the present invention, a dilator can be provided that can easily widen the diameter of a penetration-hole formed in the wall of a digestive tract or the like.

### BRIEF DESCRIPTION OF DRAWINGS

FIG. 1 is an overall view of a dilator according to a first embodiment.
FIG. 2 is a diagram showing a distal end side section of a dilator according to a second embodiment.
FIG. 3 is a diagram showing an overall view of a dilator according to a third embodiment.
FIG. 4 is a partial cross-sectional view of a distal end side section of a dilator according to a fourth embodiment.
FIG. 5 is an overall view of a dilator according to a modification.
FIG. 6 is a partial cross-sectional view of a distal end side section of a dilator according to a modification.
FIG. 7 is a partial cross-sectional view of a distal end side section of a dilator according to a modification.
FIG. 8 is a partial cross-sectional view of a distal end side section of a dilator according to a modification.
FIG. 9 is a partial cross-sectional view of a distal end side section of a dilator according to a modification.
FIG. 10 is a partial cross-sectional view of a distal end side section of a dilator according to a modification.
FIG. 11 is a partial cross-sectional view of a distal end side section of a dilator according to a modification.
FIG. 12 is a schematic view of a distal end side section of the dilator according to the first embodiment, the third embodiment, and a modification.
FIG. 13 is a schematic view showing a distal end side section of a dilator according to a modification.
FIG. 14 is a schematic view showing a distal end side section of a dilator according to a modification.
FIG. 15 is a schematic view showing a distal end side section of a dilator according to a modification.
FIG. 16 is a schematic view showing a distal end side section of a dilator according to a modification.
FIG. 17 is a diagram showing a distal end side section of a dilator according to a modification.

### DESCRIPTION OF EMBODIMENTS

The invention is defined in claim 1. Further aspects and preferred embodiments are defined in the appended claims. Aspects, embodiments and examples of the present disclosure which do not fall under the scope of the appended claims do not form part of the invention and are merely provided for illustrative purposes.

Hereinafter, embodiments of the present invention will be described with reference to the drawings. Note that the dimensions of the dilators shown in the drawings are dimensions shown for the purpose of facilitating an understanding of the technical matters, and do not correspond to the actual dimensions.

### <First Embodiment>

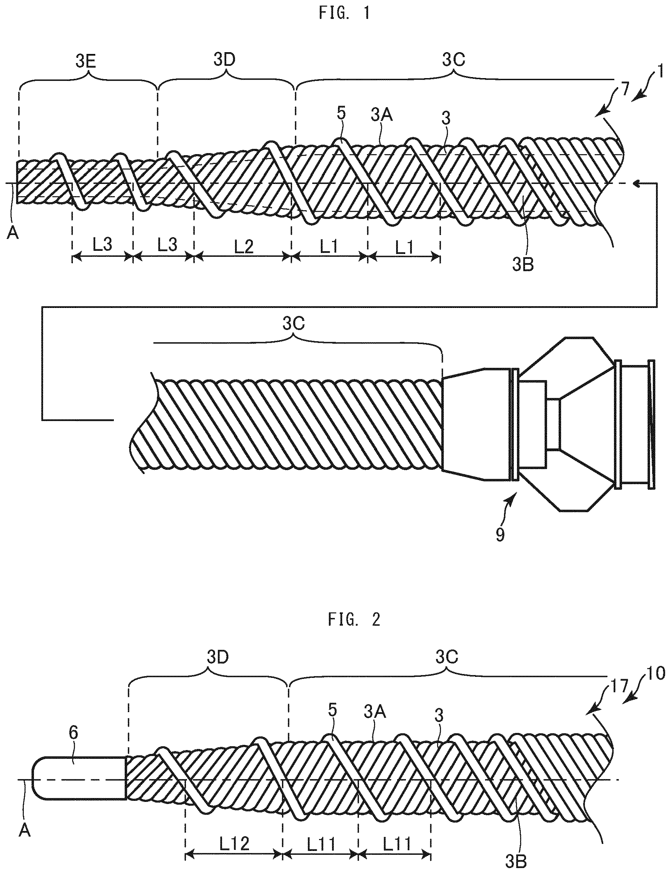

A first embodiment of the present invention will be described with reference to the drawings. FIG. 1 is an overall view of a dilator 1 according to a first embodiment of the present invention. Furthermore, in FIG. 1, the left side of the drawing is the distal end side (far side) inserted into the body, and the right side is the proximal end side (near side) operated by an operator such as a physician.

In FIG. 1, the dilator 1 includes a multilayered body 7 including a first coil 3 formed by winding a plurality of metal wires into a hollow shape, and a second coil 5 formed by winding a single metal wire onto an outer peripheral face 3A of the first coil 3 in an opposite direction (wound clockwise toward the distal end) to the first coil 3 (wound counterclockwise toward the distal end); and a hollow connector 9 connected to a proximal end of the multilayered body 7.

The wires constituting the first coil 3 and the second coil 5 are, for example, metal wires made of stainless steel or a superelastic alloy such as nickel-titanium, or are resin wires.

The first coil 3 is formed, for example, by winding ten metallic wires made of stainless steel. The first coil 3 has a hollow shape and is formed having a lumen 3B that passes through from the distal end to the proximal end. The first coil 3 includes a proximal end part 3C, a tapered part 3D, and a distal end part 3E. The first coil 3 corresponds to a shaft.

The proximal end part 3C is positioned on the proximal end side of the dilator 1, and a connector 9 is connected to the proximal end thereof. Furthermore, the proximal end part 3C has a substantially constant outer diameter from the proximal end to the distal end.

The tapered part 3D is positioned on the distal end side of the proximal end part 3C, extends from the distal end of the proximal end part 3C toward the distal end side, and has an outer diameter that decreases toward the distal end side.

The distal end part 3E is located on the distal end side of the tapered part 3D and extends from the distal end of the tapered part 3D toward the distal end side. The distal end part 3E has a substantially constant outer diameter from the proximal end to the distal end. As a result, the first coil 3, which serves as the shaft, has a hollow shape in which the outer diameter of the distal end is smaller than the outer diameter of the proximal end.

The second coil 5 is, for example, a single metal wire which is wound around onto the outer peripheral face 3A of the first coil 3 in an opposite direction (wound clockwise toward the distal end) to the first coil 3 (wound counterclockwise toward the distal end). Here, the metal wire is closely wound on the proximal end side, and is wound with spacing on the distal end side of the proximal end part 3C, the tapered part 3D, and the distal end part 3E. A spirally-arranged protruding portion that protrudes toward the exterior (from the outermost surface and outermost part of the dilator 1) is provided on the outer peripheral face 3A of the first coil 3 as a result of the section of the second coil 5 being wound with spacing. The spirally-arranged protruding portion has gaps in sections that are neighboring (metal wires that are neighboring) along the axis A of the first coil 3. The dilator 1 can also be moved forward by a rotation operation of the dilator 1 as a result of a screw action of the spirally-arranged protruding portion.

Furthermore, the pitch of sections provided on the tapered part 3D, said sections being neighboring along the axis A of the second coil 5, is configured to be larger than the pitch of sections provided on the proximal end part 3C and the distal end part 3E, said sections being neighboring along the axis A of the second coil 5. That is to say, in terms of the sections being neighboring along the axis A of the second coil 5, the pitch L1 on the distal end side of the proximal end part 3C, the pitch L2 on the tapered part 3D, and the pitch L3 on the distal end part 3E are configured such that L1, L3 < L2. Note that the pitches on the distal end side of the proximal end part 3C may be equal or different. The pitches on the tapered part 3D may be equal or different.

Furthermore, the metal wire of the second coil 5 is wound such that the amount of separation between neighboring metal wires gradually decreases toward the proximal end side of the proximal end part 3C. As a result of this configuration, the rigidity of the dilator 1 (multilayered body 7) in the axial direction can be gradually changed, and the dilator 1 can easily advance along a path even when a curved path is to be travelled.

The length of the dilator in the present embodiment and in the other embodiments described below is, for example, 2000 mm, and preferably 1600 mm to 2500 mm, the length of the distal end part 3E is, for example, 10 mm, and preferably 0 to 100 mm, and further, the length of the tapered part 3D is, for example, 30 mm, and preferably 5 to 100 mm. The inner diameter at the distal end of the first coil 3 is, for example, 0.7 mm, and preferably 0.4 to 1.0 mm, and the inner diameter at the proximal end of the first coil 3 is, for example, 1.5 mm, and preferably 1.0 to 3.0 mm. The outer diameter at the distal end of the second coil 5 is, for example, 1.84 mm, and preferably 0.8 to 3.0 mm, and the outer diameter at the proximal end of the second coil 5 is, for example, 2.64 mm, and preferably 1.4 to 5.0 mm. Furthermore, the diameter of the metal wires of the first coil 3 is, for example, 0.21 mm, and preferably 0.1 to 0.5 mm, and the diameter of the metal wires of the second coil 5 is, for example, 0.36 mm, and preferably 0.1 to 0.5 mm.

The pitches L1 and L3 of the second coil 5 on the proximal end part 3C and the distal end part 3E are, for example, 1.5 mm, the pitch L2 of the second coil 5 on the tapered part 3D is, for example, 2 mm, and the ratio between the two (L1 or L3/L2) is 0.75. The pitches L1 and L3 of the second coil 5 on the proximal end part 3C and the distal end part 3E are preferably 0.2 to 4 mm, the pitch L2 of the second coil 5 on the tapered part 3D is preferably 0.25 to 5 mm, and the ratio between the two is in a range of 0.04 to 1.

The connector 9, which is a grip part, is a part that an operator uses to push the dilator into the body or perform a rotation operation. The distal end of the connector 9 is connected to the proximal end of the first coil 3 and the proximal end of the second coil 5. The connector 9 is made of resin, and has a hollow shape having a lumen which communicates with the lumen 3B of the first coil 3.

In the dilator 1 of the present invention, a spirally-arranged protruding portion (second coil 5) that protrudes toward the exterior is provided on the outer peripheral face 3A of first coil 3, which serves as the shaft, and the spirally-arranged protruding portion has gaps in sections that are being neighboring along the axial direction of the first coil 3. This configuration not only enables the dilator to be moved forward in a conventional fashion by a pressing operation, but also to be moved forward by a rotation operation as a result of the spirally-arranged protruding portion.

Furthermore, the pitch of sections provided on the tapered part 3D, said sections being neighboring along the axis A of the second coil 5, is configured to be larger than the pitch of sections provided on the proximal end part 3C and the distal end part 3E, said sections being neighboring along the axis A of the second coil 5. As a result, when the dilator 1 is rotated, the tapered part 3D of the first coil 3 has a smaller frictional resistance with the target object (for example, a digestive tract such as the stomach, or the liver) than the proximal end part 3C and the distal end part 3E. As a result, the diameter of a penetration-hole formed in the wall of a digestive tract or the like can be easily widened, and it is possible to suppress damage to the target object which can occur due to intrusion into the target object.

Moreover, because the shaft is constituted by the first coil 3, in which a plurality of metal wires is wound in a hollow shape, the flexibility of the shaft, and the torquability by the first coil 3 can be improved. Furthermore, because the spirally-arranged protruding portion is composed of the second coil 5, in which a single metal wire is wound around onto the outer peripheral face 3A of the first coil 3, the spirally-arranged protruding portion can be easily formed, and the elasticity of the second coil 5 enables the flexibility of distal end of the dilator 1 to be ensured, and the torquability to be improved. Moreover, because the wires of the first coil 3 and the second coil 5 are wound in mutually opposite directions, even when the dilator 1 is rotated in a direction that opens the first coil 3, a force is applied in a direction that closes the second coil 5, which inhibits the first coil 3 from opening and enables the force applied to the connector 9 of the dilator 1 to be delivered to the distal end side.

Next, an example of a usage mode of the dilator will be described.

First, a target object is punctured with an introducer needle to form a penetration-hole. Then, after inserting a guide wire into a lumen of the introducer needle, the introducer needle is removed.

Next, the proximal end of the guide wire is inserted into the lumen of the dilator, and the dilator is inserted. Then, the dilator is pushed forward while rotating the shaft to expand the hole of the punctured part. At this time, the tapered portion advances due to a screw action or the like of the spirally-arranged protruding portion caused by a rotation operation of the shaft, and the penetration-hole can be smoothly expanded by the tapered part.

### <Second Embodiment>

FIG. 2 is a diagram showing a distal end side section of a dilator 10 according to a second embodiment.

Furthermore, in FIG. 2, the left side of the drawing is the distal end side (far side) inserted into the body, and the right side is the proximal end side (near side) operated by an operator such as a physician.

The dilator 10 of the present embodiment has the same basic structure as the dilator 1 of the first embodiment, and therefore, the same reference numerals are given to the same members, and the description will be omitted.

In FIG. 2, the dilator 10 includes a multilayered body 17 including a first coil 3 formed by winding a plurality of metal wires into a hollow shape, and a second coil 5 formed by winding a single metal wire onto an outer peripheral face 3A of the first coil 3 in an opposite direction (wound clockwise toward the distal end) to the first coil 3 (wound counterclockwise toward the distal end); and a hollow connector 9 connected to a proximal end of the multilayered body 17. However, the dilator 10 differs from the dilator 1 in that a tip 6 is provided instead of the distal end part 3E of the first coil 3 of the dilator 1. In the present embodiment, the first coil 3, which is provided with the tip 6 on the distal end, corresponds to the shaft.

The tip 6 is formed by pouring a brazing material (such as a silver-tin brazing material or a gold-tin brazing material) into the distal end of the first coil 3, and the shape thereof is a substantially cylindrical hollow shape. Moreover, unlike the distal end of the multilayered body 7, the surface of the tip 6 does not have an uneven shape, and is flat.

In the present embodiment, the pitch of sections provided on the tapered part 3D, said sections being neighboring along the axis A of the second coil 5, is configured to be larger than the pitch of sections provided on the proximal end part 3C, said sections being neighboring along the axis A of the second coil 5. That is to say, in terms of the sections being neighboring along the axis A of the second coil 5, the pitch L11 on the distal end side of the proximal end part 3C and the pitch L12 on the tapered part 3D are configured such that L11 < L12.

According to the dilator 10 having such a configuration, the same effects as those of the dilator 1 according to the first embodiment can be obtained. That is to say, when the dilator 10 is rotated, the tapered part 3D of the first coil 3 has a smaller frictional resistance with the target object (for example, a digestive tract such as the stomach, or the liver) than the proximal end part 3C. As a result, the diameter of a penetration-hole formed in the wall of a digestive tract or the like can be easily widened, and it is possible to suppress damage to the target object which can occur due to intrusion into the target object. Furthermore, because the tip 6 having a flat surface is connected to the distal end of the multilayered body 17, the insertability into a punctured part is further improved by first pushing the dilator with respect to the punctured part, and then pushing the dilator while applying a rotation.

### <Third Embodiment>

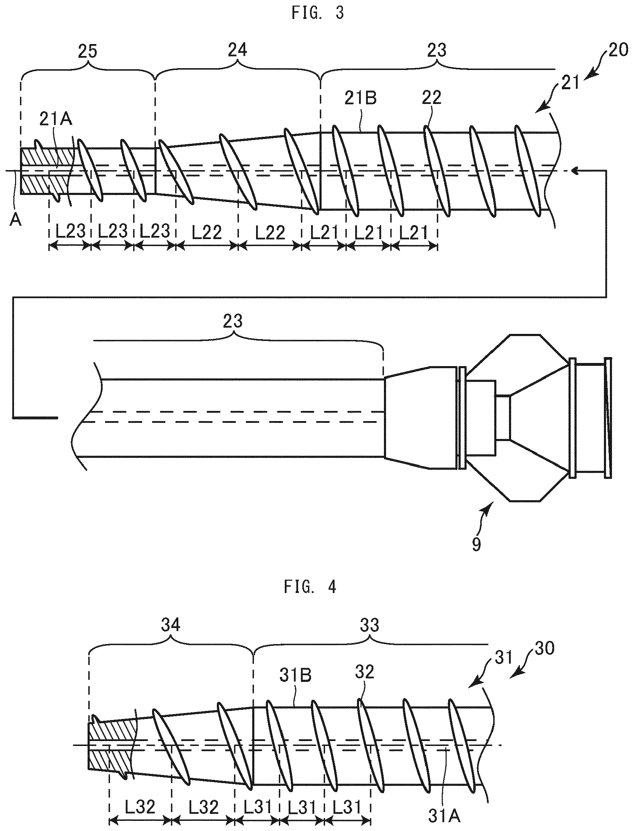

FIG. 3 is an overall view of a dilator 20 according to a third embodiment.

Furthermore, in FIG. 3, the left side of the drawing is the distal end side (far side) inserted into the body, and the right side is the proximal end side (near side) operated by an operator such as a physician.

In FIG. 3, the dilator 20 includes a shaft 21, a spirally-arranged protruding portion 22, and a connector 9 that is connected to the proximal end of the shaft 21.

The shaft 21 has a hollow shape and is formed having a lumen 21A that passes through from the distal end to the proximal end. Furthermore, the shaft 21 includes a proximal end part 23, a tapered part 24, and a distal end part 25.

The material forming the shaft 21 and the spirally-arranged protruding portion 22 is not particularly limited as long as it ensures the flexibility of the tapered part 24 and the distal end part 25 and is biocompatible, and examples include stainless steel, superelastic alloy materials such as nickel-titanium alloy, and synthetic resins such as polyvinyl chloride resins, urethane resins, polyolefin resins, polyamide resins, and fluorine resins.

The proximal end part 23 is positioned on the proximal end side of the dilator 20, and a connector 9 is connected to the proximal end thereof. Furthermore, the proximal end part 23 is provided on the proximal end side of the tapered part 24 and extends toward the proximal end side. The proximal end part 23 has a substantially constant outer diameter from the proximal end to the distal end.

The tapered part 24 is connected to the distal end of the proximal end part 23, extends from the distal end toward the distal end side, and has a shape which is tapered toward the distal end side.

The distal end part 25 is connected to the distal end of the tapered part 24 and extends from the distal end toward the distal end side. The distal end part 25 has a substantially constant outer diameter from the proximal end to the distal end. As a result, the shaft 21 has a hollow shape in which the outer diameter of the distal end is smaller than the outer diameter of the proximal end.

A spirally-arranged protruding portion 22 is provided on the outer peripheral face 21B of the shaft 21 so as to protrude toward the exterior (from the outermost surface and outermost part of the dilator 20). The spirally-arranged protruding portion 22 is provided on a distal end side section of the proximal end part 23, the tapered part 24, and the distal end part 25, and has gaps in sections that are neighboring along an axis A of the shaft 21. That is to say, sections of the spirally-arranged protruding portion 22 that are neighboring are mutually separated. The spirally-arranged protruding portion 22 is integrally formed with the shaft 21 by casting or the like.

The pitch of sections provided on the tapered part 24, said sections being neighboring along the axis A of the spirally-arranged protruding portion 22, is configured to be larger than the pitch of sections provided on the proximal end part 23 and the distal end part 25, said sections being neighboring along the axis A of the spirally-arranged protruding portion 22. That is to say, in terms of the sections being neighboring along the axis A of the spirally-arranged protruding portion 22, the pitch L21 on the proximal end part 23, the pitch L22 on the tapered part 24, and the pitch L23 on the distal end part 25 are configured such that L21, L23 < L22. Note that the pitches on the proximal end part 23 and the distal end part 25 may be equal or different. The pitches on the tapered part 24 may be equal or different.

In the dilator 20 of the present embodiment, a spirally-arranged protruding portion 22 that protrudes toward the exterior is provided on the outer peripheral face 21B of shaft 21, and the spirally-arranged protruding portion 22 has gaps in sections that are neighboring along the axis A of the shaft 21. This configuration not only enables the dilator to be moved forward in a conventional fashion by a pressing operation, but also to be moved forward by a rotation operation as a result of the spirally-arranged protruding portion 22.

Furthermore, the pitch of sections provided on the tapered part 24, said sections being neighboring along the axis A of the spirally-arranged protruding portion 22, is configured to be larger than the pitch of sections provided on the proximal end part 23 and the distal end part 25, said sections being neighboring along the axis A of the spirally-arranged protruding portion 22. As a result, when the dilator 20 is rotated, the tapered part 24 of the shaft 21 has a smaller frictional resistance with the target object (for example, a digestive tract such as the stomach, or the liver) than the proximal end part 24 and the distal end part 25. As a result, the diameter of a penetration-hole formed in the wall of a digestive tract or the like can be easily widened, and it is possible to suppress damage to the target object which can occur due to intrusion into the target object.

### <Fourth Embodiment>

FIG. 4 is a partial cross-sectional view of a distal end side section of a dilator 30 according to a fourth embodiment of the present invention. Furthermore, in FIG. 4, the left side of the drawing is the distal end side (far side) inserted into the body, and the right side is the proximal end side (near side) operated by an operator such as a physician.

In FIG. 4, the dilator 30 includes a shaft 31, a spirally-arranged protruding portion 32, and a connector 9 that is connected to the proximal end of the shaft 31 (see FIG. 3). The materials forming the shaft 31 and the spirally-arranged protruding portion 32 are the same as the materials forming the shaft 21 and the spirally-arranged protruding portion 22 of the dilator 20 of the third embodiment.

The shaft 31 has a hollow shape and is formed having a lumen 31A that passes through from the distal end to the proximal end. Furthermore, the shaft 31 includes a proximal end part 33 and a tapered part 34. The dilator 30 of the present embodiment differs from the dilator 20 of the third embodiment in that it does not have a distal end part.

The configurations of the proximal end part 33 and the tapered part 34 are the same as those of the proximal end part 23 and the tapered part 24 of the third embodiment. Furthermore, a spirally-arranged protruding portion 32 is provided on the outer peripheral face 31B of the shaft 31 so as to protrude toward the exterior (from the outermost surface and outermost part of the dilator 30). The spirally-arranged protruding portion 32 is provided on a distal end side section of the proximal end part 33, and the tapered part 34, and has gaps in sections that are neighboring along an axial direction of the shaft 31. That is to say, sections of the spirally-arranged protruding portion 32 that are neighboring are mutually separated. The spirally-arranged protruding portion 32 is integrally formed with the shaft 31 by casting or the like.

The pitch of sections provided on the tapered part 34, said sections being neighboring along the axis A of the spirally-arranged protruding portion 32, is configured to be larger than the pitch of sections provided on the proximal end part 33, said sections being neighboring along the axis A of the spirally-arranged protruding portion 32. That is to say, in terms of the sections being neighboring along the axis A of the spirally-arranged protruding portion 32, the pitch L31 on the proximal end part 33 and the pitch L32 on the tapered part 34 are configured such that L31 < L32. Note that the pitches on the proximal end part 33 may be equal or different. The pitches on the tapered part 34 may be equal or different.

In the dilator 30 of the present invention, a spirally-arranged protruding portion 32 that protrudes toward the exterior is provided on the outer peripheral face 31B of shaft 31, and the spirally-arranged protruding portion 32 has gaps in sections that are neighboring along the axis A of the shaft 31. This configuration not only enables the dilator to be moved forward in a conventional fashion by a pressing operation, but also to be moved forward by a rotation operation as a result of the spirally-arranged protruding portion 32.

Furthermore, the pitch of sections provided on the tapered part 34, said sections being neighboring along the axis A of the spirally-arranged protruding portion 32, is configured to be larger than the pitch of sections provided on the proximal end part 33, said sections being neighboring along the axis A of the spirally-arranged protruding portion 32. As a result, when the dilator 30 is rotated, the tapered part 34 of the shaft 31 has a smaller frictional resistance with the target object (for example, a digestive tract such as the stomach, or the liver) than the proximal end part 34. As a result, the diameter of a penetration-hole formed in the wall of a digestive tract or the like can be easily widened, and it is possible to suppress damage to the target object which can occur due to intrusion into the target object.

Although the embodiments of the present invention have been described above, the present invention is not limited to these embodiments, and various modifications can be made.

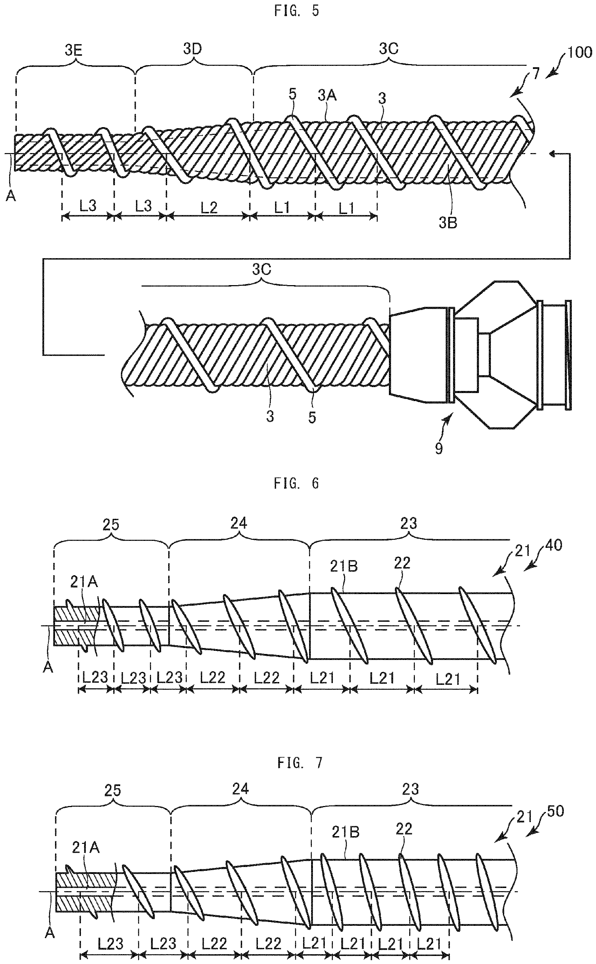

For example, as shown in FIG. 5, the dilator 1 of the first embodiment may be a dilator 100 in which the second coil 5 has gaps in sections that are neighboring along the axial direction of the first coil 3 up to the proximal end thereof.

Furthermore, in terms of the spirally-arranged protruding portion 22 of the dilator 20 of the third embodiment shown in FIG. 3, the pitch of sections provided on the tapered part 24, said sections being neighboring along the axis A of the spirally-arranged protruding portion 22, is configured to be larger than the pitch of sections provided on the proximal end part 23 and the distal end part 25, said sections being neighboring along the axis A of the spirally-arranged protruding portion 22. However, like the dilator 40 shown in FIG. 6, the pitches (L21 and L22) of sections provided on the tapered part 24 and the proximal end part 23, said sections being neighboring along the axis A of the spirally-arranged protruding portion 22, may be configured to be larger than the pitch (L23) of sections provided on the distal end part 25, said sections being neighboring along the axis A of the spirally-arranged protruding portion 22. That is to say, a configuration is possible where L23 < L21, L22. Note that the pitches on the distal end part 25 may be equal or different. The pitches on the tapered part 24 and the proximal end part 23 may be equal or different. As a result, when the dilator 40 is rotated, the tapered part 24 of the shaft 21 has a smaller frictional resistance with the target object (for example, a digestive tract such as the stomach, or the liver) than the distal end part 25. As a result, the diameter of a penetration-hole formed in the wall of a digestive tract or the like can be easily widened, and it is possible to suppress damage to the target object which can occur due to intrusion into the target object.

Furthermore, like the dilator 50 shown in FIG. 7, the pitches (L22 and L23) of sections provided on the tapered part 24 and the distal end part 25, said sections being neighboring along the axis A of the spirally-arranged protruding portion 22, may be configured to be larger than the pitch (L21) of sections provided on the proximal end part 23, said sections being neighboring along the axis A of the spirally-arranged protruding portion 22. That is to say, a configuration is possible where L21 < L23, L22. Note that the pitches on the proximal end part 23 may be equal or different. The pitches on the tapered part 24 and the distal end part 25 may be equal or different. As a result, when the dilator 50 is rotated, the tapered part 24 of the shaft 21 has a smaller frictional resistance with the target object (for example, a digestive tract such as the stomach, or the liver) than the proximal end part 23. As a result, the diameter of a penetration-hole formed in the wall of a digestive tract or the like can be easily widened, and it is possible to suppress damage to the target object which can occur due to intrusion into the target object.

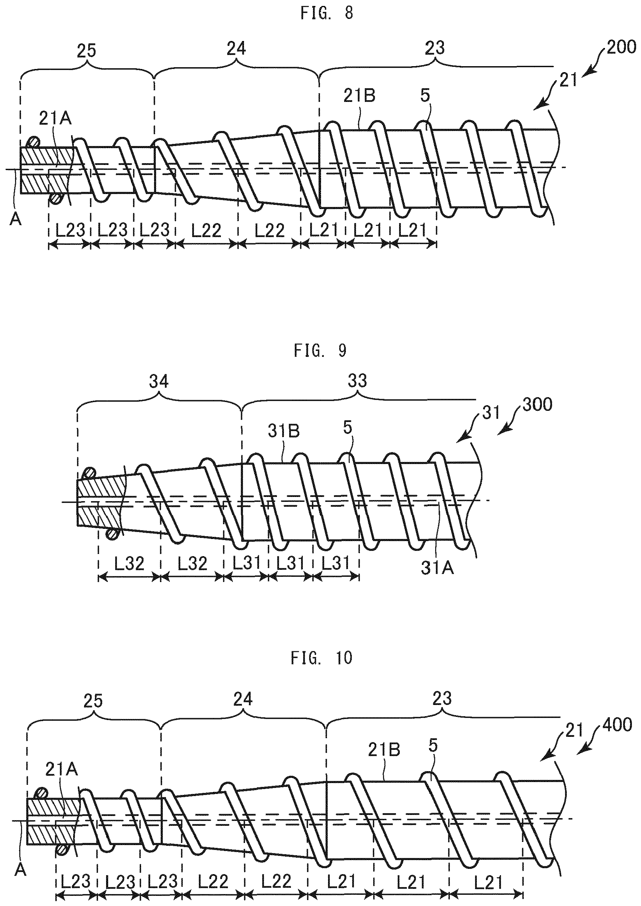

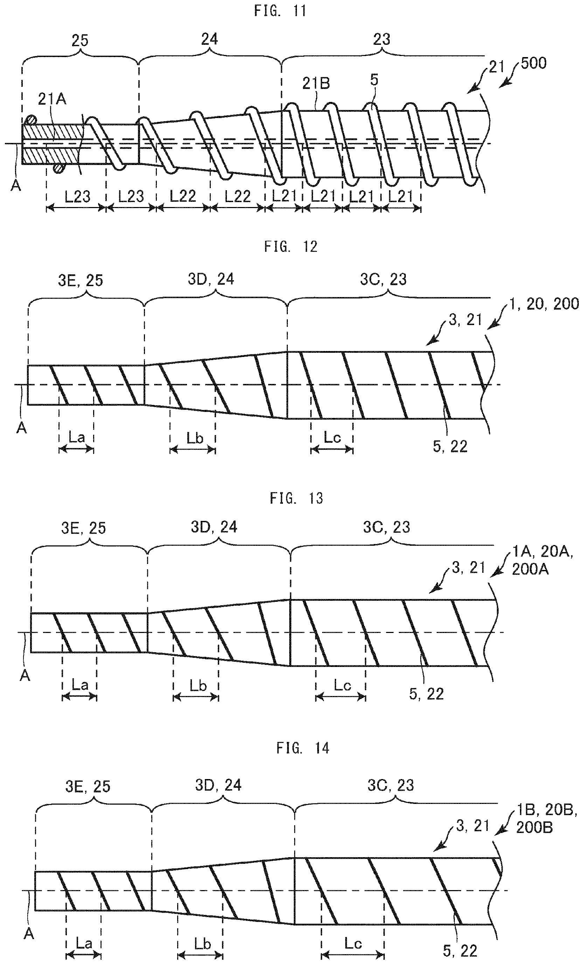

Furthermore, in the first embodiment described above, a dilator 1 was described in which the shaft and the spirally-arranged protruding portion are both constituted by a coil, and in the third and fourth embodiments and a modification of the third embodiment, dilators 20, 30, 40, and 50 were described in which the shafts 21 and 31, and the spirally-arranged protruding portions 22 and 32 are integrally formed by casting or the like. However, the dilator may be formed such, like the shafts 21 and 31, only the shaft is formed by casting, and the spirally-arranged protruding portion is constituted by a coil. That is to say, the dilator also may be a dilator 200 configured by a shaft 21 and a spirally-arranged protruding portion (second coil 5) as shown in FIG. 8, a dilator 300 configured by a shaft 31 and a spirally-arranged protruding portion (second coil 5) as shown in FIG. 9, a dilator 400 configured by a shaft 21 and a spirally-arranged protruding portion (second coil 5) as shown in FIG. 10, or a dilator 500 configured by a shaft 21 and a spirally-arranged protruding portion (second coil 5) as shown in FIG. 11.

Note that the pitches of sections, being neighboring along the axis A of the spirally-arranged protruding portion, (second coil 5) are configured in the dilator 200 of FIG. 8 such that L21, L23 < L22, are configured in the dilator 300 of FIG. 9 such that L31 < L32, are configured in the dilator 400 of FIG. 10 such that L23 < L21, L22, and are configured in the dilator 500 of FIG. 11 such that L21 < L22, L23.

Furthermore, as shown in FIG. 12, in the dilator 1 (FIG. 1), the dilator 20 (FIG. 3), and the dilator 200 (FIG. 8), the pitch of sections provided on the tapered parts 3D and 24, said sections being neighboring along the axis A of the second coil 5 and the spirally-arranged protruding portion 22, is configured to be larger than the pitch of sections provided on the proximal end parts 3C and 23 and the distal end parts 3E and 25, said sections being neighboring along the axis A of the second coil 5 and the spirally-arranged protruding portion 22. That is to say, as shown in FIG. 12, in terms of the sections neighboring along the axis A of the second coil 5 and the spirally-arranged protruding portion 22, the pitch La on the distal end parts 3E and 25, the pitch Lb on the tapered parts 3D and 24, and the pitch Lc on the distal end side of the proximal end parts 3C and 23 are configured such that La, Lc < Lb.

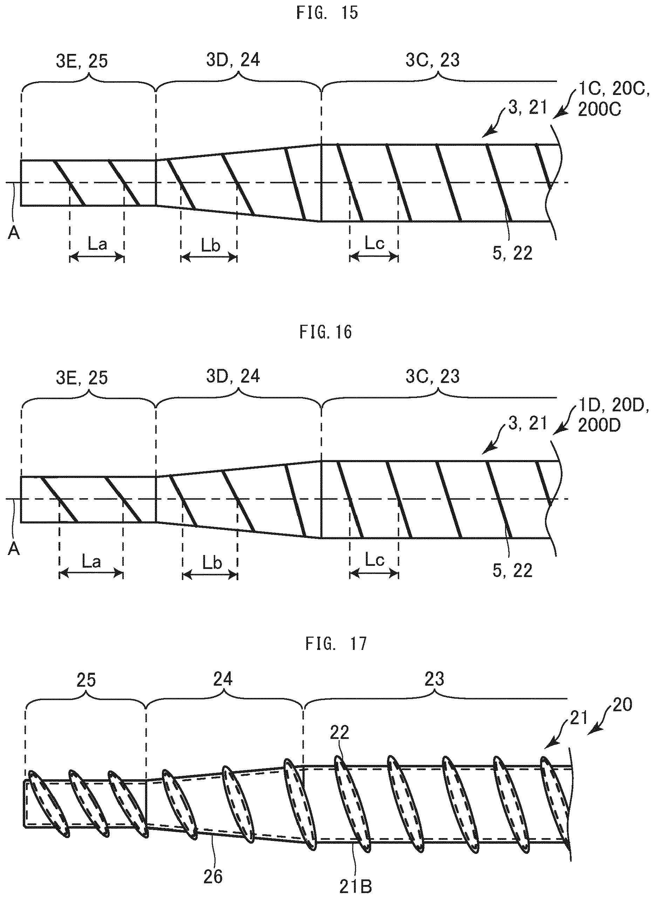

However, if the relationship La < Lb is satisfied, for example, dilators 1A, 20A, and 200A, where La < Lb = Lc (see FIG. 13), and the dilators 1B, 20B, and 200B, where La < Lb < Lc (see FIG. 14) are also possible. Furthermore, if the relationship Lc < Lb is satisfied, for example, dilators 1C, 20C, and 200C, where Lc < Lb = La (see FIG. 15), and dilators 1D, 20D, and 200D, where Lc < Lb < La (see FIG. 16) are also possible.

Note that the dilators 1A, 1B, 1C, and 1D are modifications of a dilator where the shaft and the spirally-arranged protruding portion are both configured by a coil like the dilator of FIG. 1, the dilators 20A, 20B, 20C, and 20D are modifications of a dilator where the shaft and the spirally-arranged protruding portion are both integrally formed by casting or the like the dilator of FIG. 3, and further, the dilators 200A, 200B, 200C, and 200D are modifications of a dilator where the shaft is formed by casting or the like, and the spirally-arranged protruding portion is configured by a coil like the dilator of FIG. 8.

Furthermore, in a dilator where the shaft and the spirally-arranged protruding portion are both integrally formed by casting or the like in a similar fashion to the dilator of FIG. 3, the spirally-arranged protruding portion may be provided up to the proximal end of the shaft. Moreover, in all of the dilators shown in the other diagrams, the second coil provided on the outer circumference of the shaft may or may not have gaps in sections that are neighboring along the axis of the shaft up to the proximal end thereof.

Moreover, in the embodiments above, although the first coil 3 was described as a hollow coil body formed from ten wires, the number of wires is not limited to ten, and may be one or more. In addition, in the embodiments above, although the second coil 5 was described as a hollow coil body formed from a single wire, the number of wires is not limited to one, and may be one or more.

Furthermore, although the tip 6 of the second embodiment is formed by pouring a brazing material into the distal end of the multilayered body 17, a tip 6 having a flat surface may be formed by polishing the outer circumference of the second coil 5 and/or the first coil 3 near the distal end part of the multilayered body 17.

Moreover, although the tip 6 of the second embodiment shown in FIG 2 is fixed to the distal end of the multilayered body 17, the tip 6 may be fixed to the distal end of any of the dilators shown in the other drawings, such as the distal end of the shaft 21 of the third embodiment, or the distal end of the shaft 31 of the fourth embodiment.

Furthermore, the multilayered bodies 7 and 17 of the dilators 1, 10, and 100, and the outer circumference of the spirally-arranged protruding portions 22 and 32 of the dilators 20, 30, 40, 50, 200, 300, 400, and 500 may be coated with a resin. For example, as shown in FIG. 17, the outer circumference of the shaft 21 and the spirally-arranged protruding portion 22 of the dilator 20 of the third embodiment may be coated with a resin 26. The resin 26 can improve the slidability. Also, as a result of this coating, in the embodiments where the shaft is constituted by the first coil, it is possible to inhibit living tissue from becoming trapped between the wires of the first coil (sections that are neighboring along the shaft axis of the first coil constituting the shaft), or in the embodiments where the spirally-arranged protruding portion includes the second coil, it is possible to inhibit living tissue from becoming trapped between the second coil and the shaft. When the outer circumference of the shaft 21 is coated by the resin 26, the sections of the proximal end part 23, the tapered part 24, and the distal end part 25 that are coated by the resin 26 correspond to the shaft 21, and the sections that protrude toward the exterior from the outer peripheral face 21B of the shaft 21 correspond to the spirally-arranged protruding portion 22. Examples of the resin 26 include biocompatible resin materials such as polyamide resins and fluororesins, and hydrophilic coating materials, and the thickness thereof is, for example, 0.1 to 300 µm. In addition, although the shafts 21 and 31 were integrally formed with the spirally-arranged protruding portions 22 and 32, these may be separately formed.

In the embodiments shown in FIG. 1 to FIG. 17, dilators not having a coating on the surface of the shaft were described. However, the shaft may have various coatings on the surface side (including parts between the shaft and the spirally-arranged protruding portion). Examples of the coating include a protective film (a typical example being a plating film) on the surface of the shaft, and a base film for improving the adhesion between the shaft and the spirally-arranged protruding portion.

In the embodiments shown in FIG. 1 to FIG. 17, the spirally-arranged protruding portion preferably does not constitute a blade. The dilators of the present embodiments expand a pre-formed penetration-hole in a target object (an example being the wall of a digestive tract such as a patient's stomach). Therefore, if the spirally-arranged protruding portion constitutes a blade, the living tissue on the inner surface of the penetration-hole becomes damaged.

Therefore, the cross-sectional shape of the spirally-arranged protruding portion (for example, the shape of a cross-section taken orthogonally to the helix direction of the spirally-arranged protruding portion shown in FIG. 3) preferably does not include a corner portion having an acute angle on the radially outer end part of the shaft. That is to say, the end part preferably has a part which is formed having, for example, a shape which contains a corner portion having an obtuse angle, or a curve (for example, a curve containing part of a circle or an ellipse).

### REFERENCE SIGNS LIST

1, 1A, 1B, 1C, 1D, 10, 20, 20A, 20B, 20C, 20D, 30, 40, 50, 100, 200, 200A, 200B, 200C, 200D, 300, 400, 500 Dilator
3 First coil
3A, 21B, 31B Outer peripheral face
5 Second coil
9 Connector
21, 31 Shaft
22, 32 Spirally-arranged protruding portion
3C, 23, 33 Proximal end part
3D, 24, 34 Tapered part
3E, 25 Distal end part
L1, L2, L3, L11, L12, L13, L21, L22, L23, L31, L32, La, Lb, Lc Pitch

## Claims

1. A dilator comprising:
a shaft (3; 21; 31) in a hollow shape; and
a grip portion that is provided on a proximal end of the shaft (3; 21; 31), wherein
the shaft (3; 21; 31) includes:
a tapered portion (3D; 24; 34) in which an outer diameter of a distal end is smaller than an outer diameter of a proximal end; and
either a distal end portion (3E; 25) that is provided on a distal end side of the tapered portion (24; 34) and which extends toward the distal end side and a proximal end portion (3C; 23; 33) that is provided on a proximal end side of the tapered portion (24; 34) and which extends toward the proximal end side, or the proximal end portion that is provided on the proximal end side of the tapered portion (3D; 24; 34) and which extends toward the proximal end side, without the distal end portion (3E; 25),
a spirally-arranged protruding portion (22; 32) is provided on an outer peripheral surface of the shaft (3; 21; 31),
the spirally-arranged protruding portion (22; 32) has gaps in sections that are neighboring along an axis of the shaft (3; 21; 31),
in a case where the shaft (3; 21; 31) does not include the distal end portion (3E; 25), a pitch (L2; L22; L32) of sections of the spirally-arranged protruding portion (22; 32) provided on the tapered portion (3D; 24; 34), which are neighboring along the axis, is larger than a pitch (L21; L31) of sections of the spirally-arranged protruding portion (22; 32) provided on the proximal end portion (3C; 23), which are neighboring along the axis, and
in a case where the shaft (3; 21; 31) includes the distal end portion (3E; 25), a pitch (L2; L22) of sections of the spirally-arranged protruding portion (22; 32) provided on the tapered portion (3D; 24; 34), which are neighboring along the axis, is larger than a pitch of sections of the spirally-arranged protruding portion (22; 32) provided on the distal end portion (3E; 25) or the proximal end portion (3C; 23), which are neighboring along the axis.

2. The dilator according to claim 1, wherein in the case where the shaft (3; 21; 31) includes the distal end portion, the pitch of sections of the spirally-arranged protruding portion (22; 32) provided on the tapered portion (3D; 24; 34), which are neighboring along a direction of the axis, is larger than the pitch of sections of the spirally-arranged protruding portion (22; 32) provided on the distal end portion, which are neighboring along the direction of the axis, and is also larger than the pitch of sections of the spirally-arranged protruding portion (22; 32) provided on the proximal end portion, which are neighboring along the direction of the axis.

3. The dilator according to claim 1 or 2, wherein the shaft (3; 21; 31) includes a first coil (3) having a wire wound around into a hollow shape.

4. The dilator according to any one of claims 1 to 3, wherein the spirally-arranged protruding portion (22; 32) includes a second coil (5) having a wire wound around on the outer peripheral surface of the shaft (3; 21; 31).

5. The dilator according to claim 1 or 2, wherein the shaft (3; 21; 31) includes a first coil (3) including a wire wound around in a hollow shape, and the spirally-arranged protruding portion (22; 32) includes a second coil (5) having a wire wound around on the outer peripheral surface of the shaft (3; 21; 31), and the wire of the first coil (3) and the wire of the second coil (5) are wound in mutually opposite directions.

## Patentansprüche

1. Dilatator, der Folgendes umfasst:
einen Schaft (3; 21; 31) in einer hohlen Form und
einen Griffabschnitt, der an einem proximalen Ende des Schaftes (3; 21; 31) vorgesehen ist, wobei
der Schaft (3; 21; 31) Folgendes aufweist:
einen konisch zulaufenden Abschnitt (3D; 24; 34), bei dem ein Außendurchmesser eines distalen Endes kleiner als ein Außendurchmesser eines proximalen Endes ist, und
entweder einen distalen Endabschnitt (3E; 25), der distalendseitig an dem konisch zulaufenden Abschnitt (24; 34) vorgesehen ist und der sich zu der Distalendseite hin erstreckt, und einen proximalen Endabschnitt (3C; 23; 33), der proximalendseitig an dem konisch zulaufenden Abschnitt (24; 34) vorgesehen ist und der sich zu der Proximalendseite hin erstreckt, oder den proximalen Endabschnitt, der proximalendseitig an dem konisch zulaufenden Abschnitt (3D; 24; 34) vorgesehen ist und der sich zu der Proximalendseite hin erstreckt, ohne den distalen Endabschnitt (3E; 25),
einen spiralförmig angeordneten, vorstehenden Abschnitt (22; 32), der an einer Außenumfangsfläche des Schaftes (3; 21; 31) vorgesehen ist,
wobei der spiralförmig angeordnete, vorstehende Abschnitt (22; 32) Zwischenräume in Abschnitten aufweist, die längs einer Achse des Schaftes (3; 21; 31) benachbart sind,
in einem Fall, in dem der Schaft (3; 21; 31) den distalen Endabschnitt (3E; 25) nicht aufweist, eine Teilung (L2; L22; L32) von Abschnitten des an dem konisch zulaufenden Abschnitt (3D; 24; 34) vorgesehenen spiralförmig angeordneten, vorstehenden Abschnitts (22; 32), die längs der Achse benachbart sind, größer ist als eine Teilung (L21; L31) von Abschnitten des an dem proximalen Endabschnitt (3C; 23) vorgesehenen spiralförmig angeordneten, vorstehenden Abschnitts (22; 32), die längs der Achse benachbart sind, und
in einem Fall, in dem der Schaft (3; 21; 31) den distalen Endabschnitt (3E; 25) aufweist, eine Teilung (L2; L22) von Abschnitten des an dem konisch zulaufenden Abschnitt (3D; 24; 34) vorgesehenen spiralförmig angeordneten, vorstehenden Abschnitts (22; 32), die längs der Achse benachbart sind, größer ist als eine Teilung von Abschnitten des an dem distalen Endabschnitt (3E; 25) oder dem proximalen Endabschnitt (3C; 23) vorgesehenen spiralförmig angeordneten, vorstehenden Abschnitts (22; 32), die längs der Achse benachbart sind.

2. Dilatator nach Anspruch 1, wobei in dem Fall, in dem der Schaft (3; 21; 31) den distalen Endabschnitt aufweist, die Teilung von Abschnitten des an dem konisch zulaufenden Abschnitt (3D; 24; 34) vorgesehenen spiralförmig angeordneten, vorstehenden Abschnitts (22; 32), die längs einer Richtung der Achse benachbart sind, größer ist als die Teilung von Abschnitten des am distalen Endabschnitt vorgesehenen spiralförmig angeordneten, vorstehenden Abschnitts (22; 32), die längs der Richtung der Achse benachbart sind, und auch größer ist als die Teilung von Abschnitten des am proximalen Endabschnitt vorgesehenen spiralförmig angeordneten, vorstehenden Abschnitts (22; 32), die längs der Richtung der Achse benachbart sind.

3. Dilatator nach Anspruch 1 oder 2, wobei der Schaft (3; 21; 31) eine erste Spirale (3) mit einem Draht aufweist, der zu einer hohlen Form herumgewickelt ist.

4. Dilatator nach einem der Ansprüche 1 bis 3, wobei der spiralförmig angeordnete, vorstehende Abschnitt (22; 32) eine zweite Spirale (5) mit einem Draht aufweist, der auf der Außenumfangsfläche des Schaftes (3; 21; 31) herumgewickelt ist.

5. Dilatator nach Anspruch 1 oder 2, wobei der Schaft (3; 21; 31) eine erste Spirale (3) mit einem Draht aufweist, der in einer hohlen Form herumgewickelt ist, und der spiralförmig angeordnete, vorstehende Abschnitt (22; 32) eine zweite Spirale (5) mit einem Draht aufweist, der auf der Außenumfangsfläche des Schaftes (3; 21; 31) herumgewickelt ist, und der Draht der ersten Spirale (3) und der Draht der zweiten Spirale (5) in zueinander entgegengesetzten Richtungen gewickelt sind.

## Revendications

1. Dilatateur, comprenant :
une tige (3; 21; 31) de forme creuse ; et
un tronçon de préhension qui est prévu à une extrémité proximale de la tige (3; 21; 31), la tige (3; 21; 31) comprenant :
un tronçon effilé (3D ; 24 ; 34) dans lequel un diamètre extérieur d'une extrémité distale est inférieur à un diamètre extérieur d'une extrémité proximale ; et
soit un tronçon d'extrémité distale (3E; 25) qui est prévu d'un côté d'extrémité distale du tronçon effilé (24 ; 34) et qui s'étend vers le côté d'extrémité distale, et un tronçon d'extrémité proximale (3C ; 23 ; 33) qui est prévu d'un côté d'extrémité proximale du tronçon effilé (24 ; 34) et qui s'étend vers le côté d'extrémité proximale, soit le tronçon d'extrémité proximale qui est prévu du côté d'extrémité proximale du tronçon effilé (3D ; 24 ; 34) et qui s'étend vers le côté d'extrémité proximale, sans le tronçon d'extrémité distale (3E ; 25),
un tronçon en saillie (22 ; 32) agencé en spirale qui est prévu sur une surface périphérique extérieure de la tige (3; 21; 31),
le tronçon en saillie (22 ; 32) agencé en spirale présentant des espaces dans des parties adjacentes le long d'un axe de la tige (3; 21; 31),
lorsque la tige (3 ; 21 ; 31) ne présente pas le tronçon d'extrémité distale (3E ; 25), un pas (L2 ; L22 ; L32) de parties adjacentes le long de l'axe, du tronçon en saillie (22 ; 32) agencé en spirale et prévu sur le tronçon effilé (3D ; 24 ; 34) étant supérieur à un pas (L21 ; L31) de parties adjacentes le long de l'axe, du tronçon en saillie (22 ; 32) agencé en spirale et prévu sur le tronçon d'extrémité proximale (3C ; 23), et
lorsque la tige (3 ; 21 ; 31) présente le tronçon d'extrémité distale (3E; 25), un pas (L2 ; L22) de parties adjacentes le long de l'axe, du tronçon en saillie (22 ; 32) agencé en spirale et prévu sur le tronçon effilé (3D ; 24 ; 34) étant supérieur à un pas de parties adjacentes le long de l'axe, du tronçon en saillie (22 ; 32) agencé en spirale et prévu sur le tronçon d'extrémité distale (3E ; 25) ou le tronçon d'extrémité proximale (3C ; 23).

2. Dilatateur selon la revendication 1, dans lequel, lorsque la tige (3 ; 21 ; 31) présente le tronçon d'extrémité distale, le pas de parties adjacentes selon un sens de l'axe, du tronçon en saillie (22 ; 32) agencé en spirale et prévu sur le tronçon effilé (3D ; 24 ; 34) est supérieur au pas de parties adjacentes selon un sens de l'axe, du tronçon en saillie (22 ; 32) agencé en spirale et prévu sur le tronçon d'extrémité distale, et est également supérieur au pas de parties adjacentes selon un sens de l'axe, du tronçon en saillie (22 ; 32) agencé en spirale et prévu sur le tronçon d'extrémité proximale.

3. Dilatateur selon la revendication 1 ou 2, la tige (3 ; 21 ; 31) comprenant une première bobine (3) dont un fil est enroulé selon une forme creuse.

4. Dilatateur selon l'une des revendications 1 à 3, le tronçon en saillie (22 ; 32) agencé en spirale comprenant une deuxième bobine (5) dont un fil est enroulé sur la surface périphérique extérieure de la tige (3 ; 21 ; 31).

5. Dilatateur selon la revendication 1 ou 2, la tige (3 ; 21 ; 31) comprenant une première bobine (3) dont un fil est enroulé selon une forme creuse, et le tronçon en saillie (22 ; 32) agencé en spirale comprenant une deuxième bobine (5) dont un fil est enroulé sur la surface périphérique extérieure de la tige (3 ; 21 ; 31), et le fil de la première bobine (3) et le fil de la deuxième bobine (5) étant enroulés dans des sens opposés l'un à l'autre.
